## Europäisches Patentamt
## European Patent Office
## Office européen des brevets

(11) Publication number: **0 097 741**
**B1**

(12) **EUROPEAN PATENT SPECIFICATION**

(45) Date of publication of patent specification: **26.03.86**

(51) Int. Cl.⁴: **A 61 B 5/04, A 61 B 5/00**

(21) Application number: **82303323.8**

(22) Date of filing: **25.06.82**

(54) **Bioelectric signal converter.**

(43) Date of publication of application:
**11.01.84 Bulletin 84/02**

(45) Publication of the grant of the patent:
**26.03.86 Bulletin 86/13**

(84) Designated Contracting States:
**BE DE FR GB IT LU NL**

(56) References cited:
**FR-A-2 258 671**
**US-A-3 753 433**
**US-A-3 830 227**
**US-A-3 882 277**
**US-A-4 121 573**
**US-A-4 337 377**

(73) Proprietor: **Van Riper, Wilbur E.**
**2902 Fernwood Drive**
**Santa Ana California 92706 (US)**
(73) Proprietor: **Laul, Virgil R.**
**1614 Louise Street**
**Laguna Beach California 92651 (US)**

(72) Inventor: **Van Riper, Wilbur E.**
**2902 Fernwood Drive**
**Santa Ana California 92706 (US)**
Inventor: **Laul, Virgil R.**
**1614 Louise Street**
**Laguna Beach California 92651 (US)**

(74) Representative: **Prentice, Raymond Roy**
**R.R. Prentice & Co. 34 Tavistock Street**
**London WC2E 7PB (GB)**

Courier Press, Leamington Spa, England.

## Description

This invention relates to a biologic signal transmitter unit which can be attached to or incorporated in telephonic apparatus for transmission of biological signals over the telephone network.

For ease of comprehension, the invention will be described in terms of a cardiac-signal apparatus, but this is not to be construed as a limitation since the unit according to the invention is also applicable to other uses as will be apparent from the following description.

It is well known that near miracles are being performed in the treatment of heart conditions; but it is not as well known that many serious cardiac distresses are encountered in posttreatment situations. These cardiac distresses may range from actual cardiac seizures, through relatively unimportant cardiac misfunctions, to groundless fears brought on by other unrelated conditions.

Such cardiac distresses may arise at any time or place—at home, while walking, while boating, in restaurants, at meetings, etc. Of course, a doctor should be contacted immediately; but, too often, the patient tends to wait out the cardiac distress. Some of the reasons for the waiting-out period are the desire not to bother the doctor, the possible embarrassment of reporting false symptoms, the fear of facing the actual cardiac reviews, etc. However, when a patient does contact the doctor, there is generally a need for a cardiac waveform—known as an EKG or an ECG; and this requires a rush trip to a hospital, medical office, or the like. Thus, a patient's fear of a cardiac-distress is worsened by an incipient panic situation and its attendant expense.

There are approximately 500,000 coronary deaths each year in the United States of America and fifty to seventy percent of these are classed as "sudden" deaths. "Sudden death" is defined as death occurring within twenty-four hours of the onset of acute symptoms which begin outside of a hospital.

The cause of death in most cases is due to venticular arrythmia, which if detected earlier might have been avoided.

One of the basic instruments used by a doctor in the identification and treatment of heart disease is the electrocardiograph (EKG).

The EKG detects the electric potentials generated by the action of the heart muscle. The permanent record of them is called an electrocardiogram.

Cardiographic analysis of a person with heart disease is frequently made at all stages of the disease.

When a patient is convalescing from one or a series of heart attacks or past heart surgery, the doctor may wish to obtain a daily or weekly electrcardiogram in order to check on his progress.

This usually requires that the patient go to the doctor's office or to a hospital for tests. This may be difficult, as well as expensive for a patient, particularly if he is in a physically and emotionally weakened condition.

It frequently happens that a person is stricken by a heart attack at work, at home, at a restaurant, on a boat, in an airplane or other inconvenient location. In most of these cases, an electrocardiogram could not be readily made.

Most people, even those with a history of heart disease, hesitate to call their physician at the onset of symptoms. The reasons for this are many and varied. Sometimes, they do not want to bother him, particularly if it is late at night, or on a weekend—and think it is probably nothing anyway. Many, particularly elderly patients, do not call the hospital or physician because it takes much effort to go to the doctor or to a hospital.

There are numerous prior-art apparatuses that attempt to solve the above problem by sending the patient's EKG over the telephone system. Invariably, the proposed solutions require chest electrodes that utilize a cardiac gel; they generally require "hard-wiring" between the electrodes and the apparatus; and they require a conversion apparatus that converts the cardiac signal (from the electrodes) to a cardiac sound. The conversion apparatus usually comprises a cradle for a telephone handset; and the proper cradling of the handset applies the cardiac sound to the handset mouthpiece for telephonic transmission. Attention is directed to United States Patents 3426150; 3872251 and 3872252.

While these prior-art apparatuses improve the patient's mobility, they require that he carry around numerous and relatively burdensome components.

Other proposed apparatuses suggest the use of radio transmission—which requires even more complex apparatuses.

These apparatuses have a number of objectionable features the first of which is that they require the use of a cardiac gel smeared onto the patient's chest where the electrodes are to be placed. Cardiac gels are used to reduce the "interface" electrical resistance between the patient's skin and the electrode proper, in order to encourage the flow of the minute electric current that forms the electric cardiac signal. Unfortunately, cardiac gels tend to be irritating to the skin; and a person who requires frequent applications of the cardiac gel soon develops a painful skin condition.

A second objectionable feature of the prior-art apparatuses is the use of electrodes that are intentionally small—so that they may pick up cardiac signals from precisely designated body areas—this often being desirable for precise diagnosis, because the EKG varies somewhat with the location of the electrodes. Thus, incorrect placement of the small electrodes used by the prior art apparatuses may produce an undesired EKG.

A third objectionable feature of the prior-art apparatuses is the presence of hard-wiring extending from the electrodes on the patient's chest to the conversion apparatus. Even though these wires are made as flexible as possible, they must be strong enough for repeated use. As a result, patient movement or lack of care by the attendant may pull off the small poorly-adhered electrodes.

Another objectionable feature of the prior-art apparatuses is that the patient has to carry several pieces of equipment plus electrodes and gel.

Still another objectionable feature is that the patient has to assemble the apparatus, plug in leads, put gel on leads, attach leads to the chest, set several controls on the equipment; and cradle the telephone on the equipment.

Still another objectionable feature is the need to set various switches and pushbuttons, etc.

Still another objectionable feature is the need for an experienced attendant, if the patient's condition is such that he cannot direct the activities.

A still further objectionable feature is that the patient must purchase a relatively expensive apparatus, and must carry this bulky apparatus with him wherever he goes.

Thus, despite numerous proposed prior-art apparatuses, there is still a need for an improved cardiac-signal apparatus.

An apparatus has been proposed, in French Patent Specification No. 2258671, for converting the signal emitted by an implanted pace-maker into a sound signal, the apparatus comprising an inductive signal detector 22, converting means 24 and a loudspeaker 21, wherein a telephone handset has to be brought close to the unit for transmitting the sound signal. However, this apparatus is only suitable for use in cases in which a pace-maker has been implanted in a patient's body.

It is the aim of the present invention to provide an improved biologic apparatus which is truly portable, does not require any hard-wiring between patient and apparatus and which can be made available for immediate use by an unskilled or debilitated person.

According to the invention, there is provided a biologic signal transmitter unit adapted to receive or to be incorporated in a telephone handset; said unit including converting means, comprising a sound transducer, for converting biologic signals to corresponding sound signals adapted to be transmitted over a telephone system; and means for conducting said converted sound signals to the mouthpiece of said telephone handset.

According to one embodiment of the invention, the biologic signal transmitter unit comprises an earpiece and a mouthpiece oppositely disposed to each other; a first pickup electrode fixedly and integrally disposed within said biologic signal transmitter unit interconnecting said pickup electrode and said converting means; a second pickup electrode fixedly and integrally attached within said biologic signal transmitter unit interconnecting said second pickup electrode and said converting means, said first and second electrodes being adapted to pick up said biologic signals and transmit said signals to said converting means; and said pickup electrodes being located in said earpiece and said mouthpiece portions respectively of said composite biologic signal transmitter unit. Preferably, in this embodiment, the unit is incorporated in a handset having the physical configuration of a telephone handset and comprises a housing having the earpiece and mouthpiece interconnected by an intermediate member.

In another embodiment of the invention, the unit is adapted to receive a telephone handset therein and comprises a housing having a substantially dumb-bell-shaped configuration; first and second pickup electrodes, each being adapted to pick up biologic signals and transmit said signals, said electrodes being formed on said housing and spaced from each other to substantially coincide with the respective position of the mouthpiece and earpiece of said telephone handset; said first and second pickup electrodes being integrally connected by an intermediate portion of said housing and the sound transducer being disposed in the intermediate portion of said housing and being connected to the pickup electrodes.

The pickup electrodes are desirably provided with a substantially cup-shaped configuration.

The invention will now be further described, by way of example, with reference to the drawings, in which:—

Fig. 1 is a perspective view of one embodiment of a biologic signal transmitter unit according to the invention;

Fig. 2 shows how a biologic signal transmitter unit according to the invention is used;

Fig. 3 is a perspective view of a second embodiment in the form of a composite biologic module comprising a telephone handset and a biologic signal transmitter unit;

Fig. 4 shows a pickup electrode adapted for use with the composite biologic module shown in Fig. 3; and

Fig. 5 is a circuit diagram.

In the drawings, like parts are denoted by like reference numerals.

Broadly speaking, the present invention provides a truly portable biologic signal transmitter unit which can be readily attached to a telephone handset, to form a hand-held biologic module. When the disclosed apparatus is to be used on a cardiac patient, the biologic module comprises a pair of integral, fixedly positioned, suitably spaced, pickup electrodes that are positioned to straddle the heart area. The disclosed apparatus inherently picks up an electric cardiac signal; converts it to a modulated cardiac sound; and tranmits this over the telephone system to a receiver station.

When the resultant EKG has been analyzed, the operator at the receiver station actuates an "alert" tone at the biologic module, this tone alerting the patient to use the biologic module as a standard telephone handset to receive instructions.

As indicated above, it is imperative for a cardiac patient to be able to transmit his EKG to a suitable receiver station; and this invention thus helps save the lives of people with heart trouble. The transmitting apparatus should preferably be so compact and lightweight that it may be carried at all times, and to any occasion that the patient chooses to attend.

Described is a portable, battery-operated biologic unit—comprising an electrocardiograph transmitter/receiver—that may be directly attached to the handpiece of a regular telephone. It is very easily operated at home or while travelling, and requires very little effort on the part of the patient. It clips directly onto the telephone handset, and requires no electrical leads of any kind connecting to the patient.

Heart-signal-pickup contacts are an integral part of the biologic unit; and, when the device is clipped in place, the heart signal electrodes fit over the telephone microphone and earpiece.

In order to send his EKG to his physician, hospital, or other suitable receiving center, all a patient has to do is to dial the appropriate telephone number, wait until the telephone is answered, identify himself, and indicate that he would like to send them his EKG.

The patient then would place the telephone handset directly on his bare chest or abdomen, and press a small pushbutton transmit switch on the biologic unit attached to the telephone. The EKG signal is picked up by the electrodes, is properly amplified, is filtered, and is converted to an audio tone (which is frequency modulated) and fed to a transducer. The transducer couples the signal into the microphone of the transmitting telephone handset. No hard-wiring is required between the telephone and the EKG.

When the receiving center begins to receive the transmitted EKG signal, the receivimg telephone is attached to the EKG receiving device; and the EKG may be printed-out and/or displayed on an appropriate oscilloscope or other recording device. Upon receiving sufficient information from the patient, the receiver center operator sends an "alert"-tone signal back to the patient's telephone. This tone and/or a companion-light signals the patient that he is to remove the telephone EKG from his body, and to release the transmit-pushbutton switch.

Perforations in the electrodes permit normal use of the telephone while the EKG is attached to the telephone, but is not being used to transmit heart signals.

The patient then uses the telephone in a normal manner and the receiving-station operator or physician could then tell him what action to take. The physician can tell whether or not the patient is in immediate danger. If the patient is indeed suffering from a heart attack, the physician can quickly dispatch an ambulance or paramedics to his aid, and instruct the patient or others close by as to what immediate action, if any, to take. If he is in no immediate danger, the physician may arrange for the patient to come to his office or to the hospital for a complete clinical EKG and examination.

One of the primary values of the invention in addition to the obvious one of being a potential life-saver, is that it will give the heart patient a much-needed sense of security and peace of mind. In having the new device readily available, he will know that his doctor and immediate help are only as far as the nearest telephone.

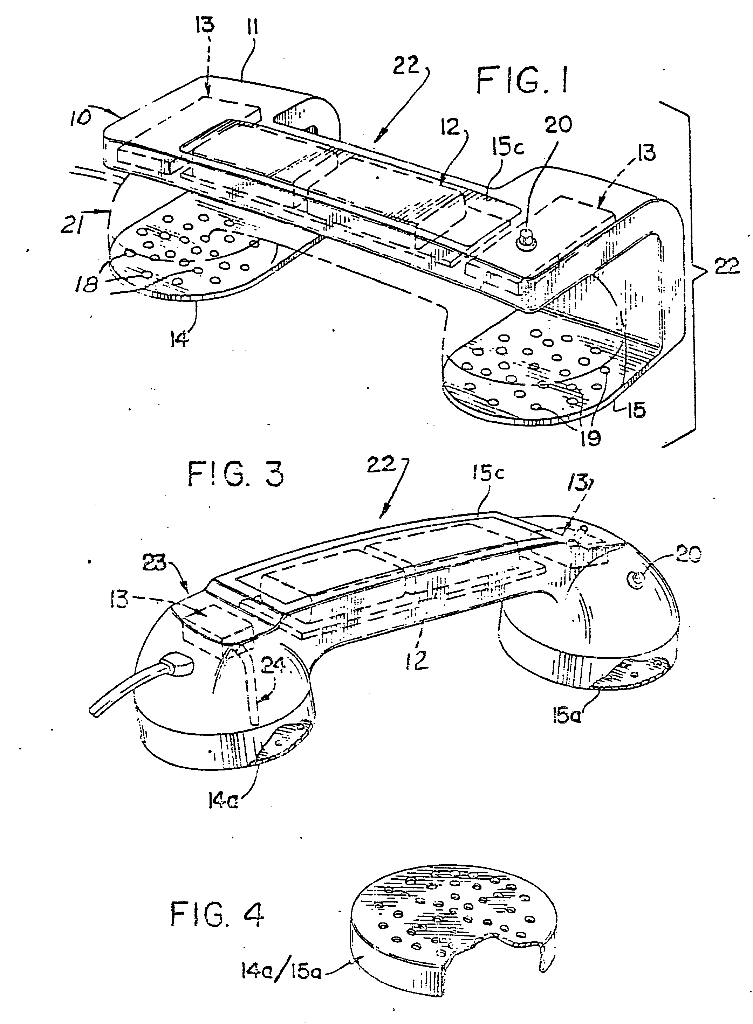

Fig. 1 shows a pictorial-and-phantom view of the biologic signal transmitter unit 10. Here, a suitable housing 11 encloses a battery operated circuitry 12 comprising a sound producer 13 and other components which will be discussed later. The biologic signal transmitter unit 10 comprises a pair of cardiac-signal pickup electrodes 14 and 15 which are fixedly positioned and suitably spaced; and are an integral part of the biologic unit 10—no hard-wiring being used to connect them into the circuitry. As indicated, pickup electrodes 14 and 15 are perforated at 18 and 19.

A master/transmit switch 20 is positioned for convenient thumb actuation.

The pickup electrodes 14 and 15 are as large as convenient—typically 64.5 to 96.8 sq. cms. (10 to 15 square inches)—or the same area as the mouthpiece and the earpiece of a telephone handset.

The pickup electrodes 14 and 15 are preferably made of stainless steel, for a number of reasons. First of all, stainless steel retains its clean surface, so that it retains its low electrical resistance for interface contact with the skin. Secondly, stainless steel is not inimical to the skin; so that its use does not cause skin sores or abrasions. Thirdly, stainless steel is strong enough so that, once formed, it retains its configuration; and it does not bend or lose its configuration.

It so happens that the optical electrode spacing for straddling the adult heart area is substantially the same as the mouthpiece/earpiece spacing of a telephone handset. Thus, the pickup electrodes 14 and 15 may be conveniently spaced so that they substantially coincide with the positions of these handset elements; and their size and shape may be substantially similar to these handset elements.

It should be noted, in passing, that the disclosed biologic unit may be used for children and for animals—in the latter case care must be taken to ensure that the pickup electrodes are in actual contact with the skin.

As indicated in Fig. 1, the biologic unit 10 can be attached to a telephone handset 21.

Here, suitable means—such as springs, the pickup electrodes themselves, resilient clips or bands, Velcro, or the like—attach the biologic unit 10 to the handset 21.

It should be noted that the biologic unit 10 is dumb-bell-shaped, and has a physical configuration which is similar to and compatible with the physical configuration of the telephone handset 21, the physical configuration of the unit 10 being defined by the oppositely disposed pickup electrodes 14 and 15 which are integrally interconnected by an intermediate portion of the housing 11, the electrodes 14 and 15 being positioned adjacent the respective mouthpiece and earpiece of the telephone handset 21; and that, when they are attached, the biologic unit 10 and the handset 21 form a single compact biologic module 22 which is as easily hand held as a telephone handset by itself.

The sound producer 13 of the biologic unit 10 is preferably positioned in proximal relation to the mouthpiece of the telephone handset 21.

It has been previously pointed out that the subject biologic unit was to be described in terms of a cardiac problem; but that it was also useful in other situations.

For this reason, the phrase "receiver station" will be used to designate any station which is adapted to receive and to act upon the telephonically received biologic information.

Fig. 2 indicates how the disclosed biologic apparatus is used. In use, the telephone is dialled to connect it with a receiver station; the reason for the call is stated—as by use of a code word; and the patient is identified. The patient is meanwhile bearing his chest.

The telephone handset 21 and the biologic unit 10 are attached, as discussed above, to form the biologic module 22; and the biologic module 22 is positioned so that the pickup electrodes 14 and 15 straddle the heart area of the patient's chest, and pick up the electric cardiac signal.

When the master/transmit switch 20 is actuated, the circuitry 12 converts the electric cardiac signal from the pickup electrodes to a modulated cardiac sound at the sound producer 13, the modulated cardiac sound having audio characteristics that vary with corresponding characteristics of the EKG. The cardiac sound enters the mouthpiece of the telephone handset 21, and is transmitted over the telephone system to the receiver station. Here, the transmitted telephone signal is transformed to a visual display and/or to a printout, and may be analyzed and/or computer-compared with previous data.

When the receiver station has completed its diagnosis, its operator transmits an "alerting tone" back to the patient's handset 21 that is still attached to the biologic unit 10; and this alerting tone alerts the patient that the receiver station desires to talk with him. The patient thereupon releases the master/transmit switch 20, and raises the entire biologic module 22—unit 10 and handset 21—to his ear and mouth. Because of the perforations in the pickup electrodes 14 and 15, the patient may now converse, without loss of time, with the personnel at the receiver station. The patient is now advised about the next procedure—be it a call to the doctor, administration of a predetermined medication, or a trip to the hospital.

In some cases—such as when a patient spends most of his time at a given location, or a location (i.e. a nursing home) where there are a number of patients—it may be desirable to leave the biologic signal transmitter unit 10 more or less permanently attached to the telephone handset 21.

Alternatively, it may be desirable to incorporate the biologic signal transmitter unit into a modified telephone handset 23—as indicated in Fig. 3—which can be permanently connected to the telephone base, or may be plugable into a suitable telephone receptacle.

In a modified handset such as indicated at 23 it may be desirable to incorporate pickup electrodes 14A and 15A (Fig. 4) directly into the handset 23; and it may further be desirable to use a "sound tube" 24 to conduct sound from a differently positioned sound transducer to the mouthpiece portion of the handset.

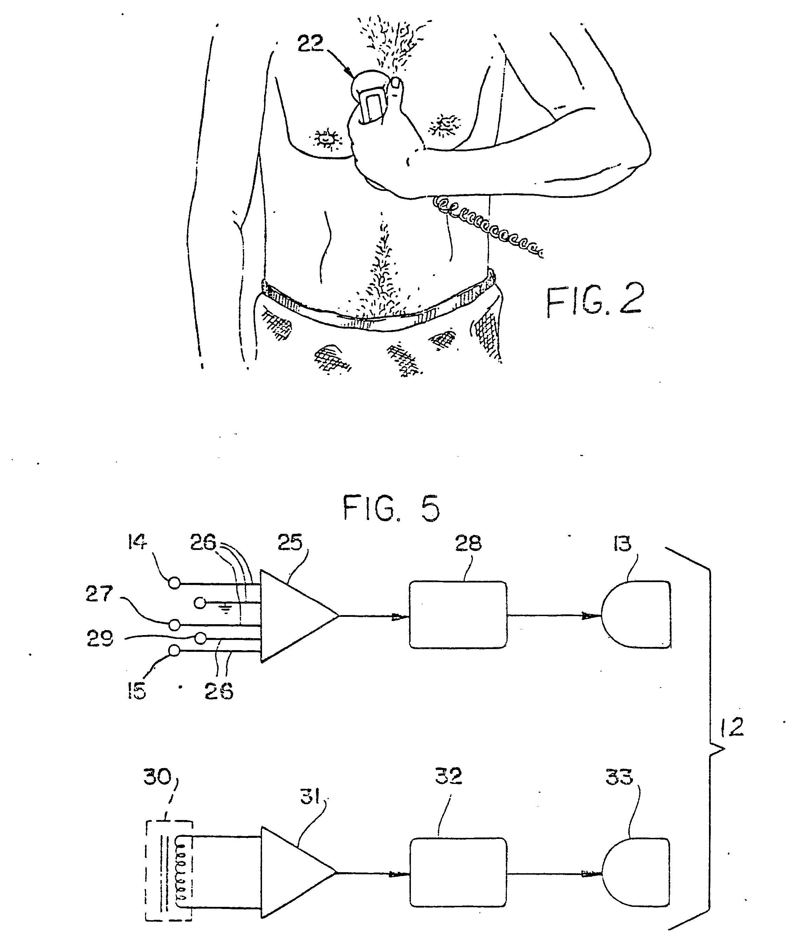

Fig. 5 shows a partial block diagram of circuitry 12 for practising the present invention. As shown in Fig. 5, the circuitry 12 consists of two pickup electrodes 14 and 15, the electric cardiac signal existing between them being applied to a differential amplifier 25 which is A.C. coupled to the electrodes in order to minimize the D.C. galvanic voltage developed as a result of the skin/electrode interface.

Attention is directed to the fact that the "connecting wires" 26 of Fig. 5 are only symbolic; the electrodes 14 and 15 are directly connected to the amplifier 25 without the use of exterior hard-wires.

If desired, a third or reference electrode 27 may be incorporated into the biologic signal transmitter unit in order to improve the signal/noise ratio; the third electrode—if used—functions as a reference for the circuitry and for the patient.

The output of the differential amplifier is applied to a voltage-to-frequency converter 28 that converts electric cardiac-signal variations (which comprise minute voltage fluctuations) to a frequency modulated electrical signal wherein the frequency fluctuations correspond to the voltage fluctuations of the original cardiac signal. The voltage-to-frequency converter 28 may comprise—for example—a two kilohertz oscillator whose two kilohertz signal is modulated by the fluctuating frequency of the cardiac signal. The unit 28 may further comprise bandpass limiters, amplitude limiters, and the like, in order to assure a frequency modulated signal that is readily handled by the telephone system.

The output of the voltage-to-frequency converter 28 is applied to the sound transducer 13 which changes the frequency modulated electric signal to a varying-frequency two kilohertz FM signal which is acoustically coupled to the telephone-handset mouthpiece (not shown in Fig. 5).

Thus, the electric cardiac signal—as picked up by the electrodes 14 and 15—eventuates into a frequency modulated two-kilohertz cardiac signal which can be well handled by the telephone system; and this telephonic signal is transmitted to the receiver station where it is recorded and/or analyzed, etc.

Alternatively, the cardiac signal may be transmitted over a radiotelephone, a citizens-band transmitter, or any other form of transmission equipment.

It was pointed out above that, while the present invention was to be presented in terms of a cardiac patient, the apparatus may be used for other purposes.

Alternatively, the input may come from another suitable sensor 29.

As indicated above, the receiver station produces an alert signal that is used to alert the patient that the EKG has been analyzed; and that the patient is to remove the biologic module from his chest, and to use the still-attached telephone handset for a normal telephonic conversation.

The receiver station may produce the alert signal by any one of a number of circuits, such as a resonant or a frequency sensitive circuit that picks up a suitable signal—typically one kilohertz—from the many frequencies present at the receiver station handset. Alternatively, it may take the form of an independent oscillator. Other suitable circuits are known.

At this time the biologic module is being held against the chest area of the patient; and it is quite possible that the alert sound may be so muffled that the patient would not hear or recognize it. Therefore, the biologic unit comprises circuitry for detecting and amplifying the alert signal from the receiver station, and for producing a loud alert sound that cannot be missed. Such a circuit is indicated in Fig. 5, wherein a pickup device 30 picks up the alert signal from the receiver station; and applies this signal to an amplifier 31, and—through a suitable filter 32—to an audio transducer 33 that produces a loud audible alert sound.

If desired, the alert sound may be replaced by, or accompanied by, a light that serves as an alert light.

The use of the biologic apparatus is described in terms of cardiac problems; but the unit may also be used to transmit blood pressure, EMG, EEC, or other signals.

In use, the biologic unit is attached to a telephone handset so that the perforated pickup electrodes cover the ear and mouthpiece portions of the telephone handset. The patient "contacts" the reference electrodes when he grasps the biologic module.

After the patient dials his physician or other facility equipped with an appropriate receiver, he identifies himself and indicates that he has some problem—such as chest pains, etc. The receiving center turns on their equipment, and tells the patient to send his EKG.

The patient does this by placing the biologic module directly on his bare chest or abdomen. He then depresses the single push-button switch which activates the transmitter and feed back receiver. If the patient has made proper contact with his body, his EKG signal is then being sent over the telephone system.

After the physical and/or receiving-center personnel have recorded enough information, they send back an alert signal over the telephone line; this signal is picked up by the biologic module and causes it to emit an audible signal and/or a flashing light signal. This tells the patient to release the pushbutton switch; to remove the biologic module from his chest; and to use the telephone in a normal manner to receive further instructions.

The patient does not have to remove the biologic unit from the telephone handset in order to use the telephone normally because the pickup electrodes are perforated, and thus permit normal telephone use.

In some cases, such as when the patient is bedridden or spends most of his time at a given location, it may be desirable to have the biologic module permanently attached to the telephone.

In other permanent installations, such as inside a hospital or nursing home, the biologic unit can be built right into the telephone handset or base of the telephone, thus forming a fixed installation.

The advantages of a biologic signal transmitter unit according to the invention are that it is very easy to use, and has minimal preparatory requirements. A patient having the slightest distress symptoms can easily and quickly transmit his EKG or other biologic signals to a receiver station. The unit is small—typically about 20 cm (8 ins.) long, 5 cm (2 ins.) wide, and 2.5 cm (1 in.) high so that it can fit into a purse, handbag, briefcase, attache case, or the like. It is also very lightweight—typically 310 gms. (11 oz.).

It is very unobtrusive, and may be carried anywhere and can be used anywhere that there is a telephone handset—in an airplane, in a boat, etc.; and this includes practically anywhere in the world. Its very presence reassures the patient.

Another important advantage is that an inexperienced person can use it successfully the very first time. A small label may provide the desired telephone number, and a picture of how it is attached and used.

As indicated above, the large stainless-steel pickup electrodes facilitate the use by an inexperienced person. For example, if this person should miss the optimal chest locations for the electrodes, the large-size electrode still provides a cardiac signal of useful amplitude and waveform. Moreover, if the inexperienced person should happen to reverse the locations of the two pickeup electrodes, the only effect would be to transmit an inverted EKG; and the personnel at the receiver station would recognize this immediately.

The disclosed biologic signal transmitter unit is completely self-contained and does not need any external connections such as hard-wiring to the patient, or electrical wiring to a power socket. In the permanent integral embodiment, the unit may be powered by the telephone line. Moreover, the disclosed apparatus does not have any switches or pushbuttons that may be inadvertently mis-set.

This invention is not intended to replace a regular clinical EKG as obtained by a physician. The main purpose of the device is to enable a patient to send a simple EKG to the hospital or physician for a quick analysis, and to do it very easily.

This invention will alleviate objections to prior-art devices, for patients can now send their EKGs to the hospital as easily as picking up their telephone. The unit may be taken by the patient on trips, in motor vehicles, on aeroplanes or anywhere else—because a doctor is as close as the nearest telephone.

The majority of cardiac arrhythmias occur either at work or at home. The new biologic apparatus is packaged into a portable form, which may be readily attached to a standard telephone handpiece, and may be carried by the patient wherever he goes.

It may, alternatively, be embodied as an integral part of a telephone or telephone-system handpiece. Or, it may be incorporated into almost any kind of radio-telephone, citizens-band transmitter or any other form of data-transmission equipment.

Another advantage of this invention is to simplify the transmission of biomedical signals (such as EKG signals) by untrained persons, even though they may be under undue stress and strain at the time of the transmission.

One of the principle advantages of the disclosed device is the elimination of the need for patient lead-wires of any kind.

For people undergoing a cardiac arrhythmia, it may be impossible to perform—particularly on very short notice while under the stress and strain of the attack. The present invention provides simplicity and ease-of-use under these conditions.

The extra large electrodes on the biologic signal transmitter unit according to this invention provide a very large skin-contact area, thus reducing the total skin resistance. This particular feature makes contact jelly for the electrodes unnecessary.

In use, the patient places the telephone handpiece with the new device properly attached, against his chest and depresses the pushbutton switch on top of the unit. As long as the switch is held depressed—and the unit is held firmly on the chest or abdomen—an EKG signal will continue to be transmitted. It is not necessary that the biologic signal transmitter unit be removed from the telephone handset when it is not being used to transmit EKG signals.

## Claims

1. A biologic signal transmitter unit adapted to receive or to be incorporated in a telephone handset; said unit including converting means, comprising a sound transducer, for converting biologic signals to corresponding sound signals adapted to be transmitted over a telephone system; and means for conducting said converted sound signals to the mouthpiece of said telephone handset.

2. A biologic signal transmitter unit according to claim 1 and comprising an earpiece and a mouthpiece oppositely disposed to each other; a first pickup electrode fixedly and integrally disposed within said biologic signal transmitter unit interconnecting said pickup electrode and said converting means; a second pickup electrode fixedly and integrally attached within said biologic signal transmitter unit interconnecting said second pickup electrode and said converting means, said first and second electrodes being adapted to pick up said biologic signals and transmit said signals to said converting means; and said pickup electrodes being located in said earpiece and said mouthpiece portions respectively of said composite biologic signal transmitter unit.

3. A biologic signal transmitter unit according to claim 2, wherein the unit is incorporated in a handset having the physical configuration of a telephone handset and comprises a housing having the earpiece and mouthpiece interconnected by an intermediate member.

4. A biologic signal transmitter unit according to claim 1, wherein the unit is adapted to receive a telephone handset therein and comprises a housing having a substantially dumb-bell-shaped configuration; first and second pickup electrodes, each being adapted to pick up biologic signals and transmit said signals, said electrodes being formed on said housing and spaced from each other to substantially coincide with the respective position of the mouthpiece and earpiece of said telephone handset; said first and second pickup electrodes being integrally connected by an intermediate portion of said housing and the sound transducer being disposed in the intermediate portion of said housing and being connected to the pickup electrodes.

5. A biologic signal transmitter unit according to any one of claims 2 to 4, wherein said pickup electrodes are perforated.

6. A biologic signal transmitter unit according to any one of claims 2 to 5, including means for causing said biologic unit to produce an alert tone, said alert tone producing means comprising pickup means for sensing an alert signal, and sound-transducer means for transducing said amplified alert signal to a loud alert sound.

7. A biologic signal transmitter unit according to any one of claims 2 to 6, including a third pickup electrode fixedly and integrally attached within said biologic unit to improve the signal/noise ratio to said converting means.

8. A biologic signal transmitter unit according to any one of claims 2 to 7, wherein the first and second pickup electrodes are provided with a substantially cup-shaped configuration.

9. A biologic signal transmitter unit according to any preceding claims, including battery-operated circuitry.

## Revendications

1. Unité transmettrice de signaux biologiques conçue pour recevoir un combiné téléphonique ou pour y être incorporée; cette unité comprenant des moyens de conversion comprenant un transducteur sonique pour convertir des signaux biologiques en signaux sonores correspondants propres à être transmis sur un réseau téléphonique; et des moyens pour conduire ces signaux sonores convertis à l'embouchure du combiné téléphonique.

2. Unité transmettrice de signaux biologiques selon la revendication 1, et comprenant un pavillon et une embouchure disposés à l'opposé l'un de l'autre; une première électrode de captage disposée de façon fixe et solidairement à l'intérieur de l'unité transmettrice de signaux biologiques reliant cette électrode de captage et les moyens de conversion; une deuxième èlectrode de captage attachée de façon fixed et solidairement à l'intérieur de l'unité transmettrice de signaux biologiques reliant cette deuxième électrode de captage et les moyens de conversion, les première et deuxième électrodes étant conçues pour capter les signaux biologiques et transmettre ces signaux aux moyens de conversion; et les électrodes de captage étant situées respectivement dans les parties de pavillon et d'embouchure de l'unité composite transmettrice de signaux biologiques.

3. Unité transmettrice de signaux biologiques selon la revendication 2, dans laquelle l'unité est incorporée à un combiné ayant la configuration physique d'un combiné téléphonique et comprenant une enveloppe présentant le pavillon et l'embouchure reliés entre eux par un élément intermédiaire.

4. Unité transmettrice de signaux biologiques selon la revendication 1, dans laquelle l'unité est conçue pour recevoir intérieurement un combiné téléphonique et comprend une enveloppe ayant une configuration pratiquement en forme d'haltère; une première et une deuxième électrodes de captage, chacune étant conçue pour capter des signaux biologiques et transmettre ces signaux, les électrodes étant formées sur l'enveloppe et espacées l'une de l'autre de manière à coïncider pratiquement avec la partie respective de l'embouchure et du pavillon du combiné téléphonique; les première et deuxième électrodes de captage étant reliées solidairement par une partié intermédiaire de l'enveloppe et le transducteur sonique étant disposé dans la partie intermédiaire de l'enveloppe et étant relié aux électrodes de captage.

5. Unité transmettrice de signaux biologiques selon l'une quelconque des revendications 2 à 4, dans laquelle les électrodes de captage sont perforées.

6. Unité transmettrice de signaux biologiques selon l'une quelconque des revendications 2 à 5, comprenant des moyens pour faire en sorte que l'unité biologique engendre un son d'avertissement, ces moyens générateurs de son d'avertissement comprenant des moyens de captage pour détecter un signal d'avertissement, des moyens d'amplification pour amplifier ce signal d'avertissement détecté et des transducteurs soniques pour convertir ce signal d'avertissement amplifié en un son d'avertissement puissant.

7. Unité transmettrice de signaux biologiques selon l'une quelconque des revendications 2 à 6, comprenant une troisième électrode de captage attachée de façon fixe et solidairement à l'intérieur de l'unité biologique pour améliorer le rapport signal/bruit des moyens de conversion.

8. Unité transmettrice de signaux biologiques selon l'une quelconque des revendications 2 à 7, dans laquelle les première et deuxième électrodes de captage présentent une configuration pratiquement en forme de cuvette.

9. Unité transmettrice de signaux biologiques selon l'une quelconque des revendications précédentes, comprenant un ensemble de circuits actionné par batterie.

**Patentansprüche ·**

.1. Biologische Signalübertragereinheit zue Aufnahme eines Telefonhörers (Telefonsprechzeugs) oder zum Einbau in einen . Telefonhörer, die Umformereinrichtungen einschließlich eines Schallwandlers zum Umwandeln biologischer Signale in entsprechende zur Übertragung in einem Telefonsystem geeignete Schallsignale sowie Einrichtungen zur Weiterleitung der umgewandelten Schallsignale zum Mundstück des Telefonhörers enthält.

2. Biologische Signalübertragereinheit nach Anspruch 1 unter Verwendung einer Hörmuschel und eines entgegengesetzt angeordneten Mundstückes, bei der sich eine erste Aufnehmerelektrode ortsfest und integral innerhalb der biologischen Signalübertragereinheit . befindet, welche die Aufnehmerelektrode und die Umformereinrichtungen miteinander verbindet, bei der sich eine zweite Aufnehmerelektrode ortsfest und integral in der biologischen Signalübertragereinheit befindet, welche die zweite Aufnehmerelektrode und die Umformereinrichtungen miteinander verbindet, wobei die ersten und zweiten Elektroden zur Aufnahme der biologischen Signale und zu deren Weiterleitung zu den Umformereinrichtungen vorgesehen sind, und bei der die Aufnahmerelektroden in den Hörmuschel- bzw. Mundstückabschnitten der zusammengesetzten biologischen Signalübertragereinheit untergebracht sind.

3. Biologische Signalübertragereinheit nach Anspruch 2, die in einem Handgerät in Form eines Telefonhörers untergebracht ist, das ein Gehäuse mit einer Hörmuschel und einem Mundstück umfaßt, die durch ein Zwischenglied miteinander verbunden sind.

4. Biologische Signalübertragereinheit nach Anspruch 1, die zur Aufnahme eines Telefonhörers dient und ein ungefähr hantelförmiges Gehäuse aufweist, bei der erste und zweite Aufnehmerelektroden zum Aufnehmen und Übertragen biologischer Signale vorgesehen und an dem Gehäuse angeformt sowie voneinander soweit entfernt angeordnet sind, daß sie im wesentlichen mit der entsprechenden Lage von Mundstück und Hörmuschel des Telefonhandhörers übereinstimmen, bei der die beiden Aufnehmerelektroden durch einen Zwischenabschnitt des Gehäuses einstückig miteinander verbunden sind, und bei der der Schallwandler in dem Zwischenabschnitt des Gehäuses untergebracht und an die Aufnehmerelektroden angeschlossen ist.

5. Biologische Signalübertragereinheit nach einem der Ansprüche 1 bis 4, bei dem die Aufnehmerelektroden perforiert sind.

6. Biologische Signalübertragereinheit nach einem der Ansprüche 2 bis 5, mit innerhalb der Einheit untergebrachten Einrichtungen zur Erzeugung eines Alarmtones, die Aufnehmereinrichtungen zum Erfassen des Alarmsignales, Verstärkereinrichtungen zur Verstärkung des ermittelten Alarmsignales und Schallwandlermittel zur Umwandlung des verstärkten Alarmsignales in einen lauten Alarmsignalton umfassen.

7. Biologische Signalübertragereinheit nach einem der Ansprüche 1 bis 6 mit einer dritten Aufnehmerelektrode, die fest und integral innerhalb der Einheit untergebracht ist, um das Verhältnis Signal/Störungsrauschen der Umwandlereinrichtungen zu verbessern.

8. Biologische Signalübertragereinheit nach einem der Ansprüche 2 bis 7, bei der die ersten und zweiten Aufnehmerelektroden eines im wesentlichen schalenförmige Ausgestaltung aufweisen.

9. Biologische Signalübertragereinheit nach einem der vorhergehenden Ansprüche, die eine batteriebetriebene elektrische Schaltung enthält.

FIG. 1

FIG. 3

FIG. 4

FIG. 2

FIG. 5